# EUROPEAN PATENT APPLICATION

(11) **EP 2 113 772 A2**
(43) Date of publication of application: **04.11.2009**
(21) Application number: 08008275.3
(22) Date of filing: 30.04.2008
(51) Int. Cl.: G01N 33/50, G01N 33/68

(54) **Induction of aging phenotypes**

(71) Applicant: Ruprecht-Karls-Universität Heidelberg, 69047 Heidelberg (DE)
(72) Inventor: Grosshans, Jörg, 69120 Heidelberg (DE); Brandt, Annely, 69123 Heidelberg (DE)
(74) Representative: Elbel, Michaela

(57) **Abstract**

The present invention concerns a method for screening an age-related candidate substance or an age-related candidate gene, an assay for screening age-related candidate substances or age-related candidate genes and the use of the assay for screening purposes. Furthermore, the invention relates to nucleic acids encoding certain age-related polypeptides.

## Description

### Field of the invention

The present invention concerns the technical field of screening age-related candidate substances and age-related candidate genes. Furthermore, the invention relates to a nucleic acid encoding certain age-related polypeptides.

### Background of the invention

Aging is generally characterized by a declining ability to respond to stress and an increasing homeostatic imbalance and an increasing risk of aging-associated degenerative diseases. In aging-associated degenerative diseases, like Alzheimer's disease, amyotrophic lateral sclerosis, cancer and Parkinson's disease, function or structure of affected tissues progressively deteriorates pathologically or due to normal bodily wear over the time. Accelerated aging diseases, like Hutchinson-Gilford Progeria syndrome (HGPS), Wemer's syndrome, Cockayne's syndrome or xeroderma pigmentosum, are conditions of childhood and youth indicated by premature aging symptoms. HGPS is a fatal disease caused by a mutation of lamin A gene on chromosome 1. Lamin A is a scaffolding protein located around the edge of the nucleus that helps organizing nuclear processes, such as RNA and DNA synthesis. In HGPS the enzymatic recognition site that is required for cleaving prelamin A to lamin A is mutated because of a splicing defect. Therefore, lamin A cannot be produced, and prelamin A builds up on the nuclear membrane causing a characteristic nuclear blebbing. This dysfunction results in premature aging symptoms indicated by dysmorphic nuclear shape, increased DNA damage and down-regulation of some nuclear proteins (Moulson et al., 2007; Scaffidi and Misteli, 2006).

The process of aging is complex, and may derive from a variety of different mechanisms. Traditionally, theories that explain senescence have generally been divided between the programmed and stochastic theories of aging. Programmed theories imply that aging is regulated by biological clocks operating throughout the life span. This regulation would depend on changes in gene expression that affect systems responsible for maintenance, repair and defense responses. Stochastic theories blame environmental impacts on living organisms as the cause of aging, wherein cumulative damages at various levels are induced to DNA, tissues and cells by oxygen radicals and cross-linking. Scientific evidence suggests that cellular aging evolved in certain species as a mechanism to prevent the onset of cancer. If cells have built-in limits to how many times they can replicate, they must somehow overcome these limits before they can spread indefinitely.

Life extension movement continues to grow rapidly in popularity and momentum among scientists and general public. Thus, there is a great demand for treatments slowing down aging processes to improve health and to maintain youthful vigor, but also to cure accelerated aging diseases. Strategies for life extension by nutritional supplements, growth hormones, and substances, like resveratrol and lipoic acid, are dubious in efficiency and side effects.

To develop potential anti-aging therapies a test system is necessary that allows displaying and manipulating an aging phenotype. Thereby, it would be possible to detect the effectiveness of age-related substances and to investigate their mechanisms and side effects. Currently, it is possible to use cells of elderly humans or HGPS patients in primary cell cultures to study cellular aging (Scaffidi and Misteli, 2006). However, in these cell cultures aging phenotypes, like changes in nuclear morphology, chromatin or DNA damage do not occur until numerous cell cycles are passed, which is tedious and cost-intensive. Unfortunately, cells of these primary cultures are heterogeneous in reference to their aging phenotype, which make experimental data incomparable. Another disadvantage of primary cell cultures in senescence studies is that they can only be cultivated temporarily, because they are not immortalized.

Model organisms for studying aging phenomena should be short-living to be practicable in reference to time and cost savings. Bauer et al. (2004) developed an assay in *Drosophila melanogaster* that accelerates the ability to evaluate potential anti-aging therapies. Coupling the expression of an age-dependent molecular biomarker to a lethal toxin reduces the time needed to perform lifespan studies by 80%. However, in this model system aging cannot be induced user-defined.

Therefore, there is a great need for a cost and labor conscious method to screen for age-related substances that affect life span or accelerated aging diseases, wherein aging can be reproducibly induced.

The solution to this problem is achieved by the embodiments of the present invention characterized by the claims, and described further below.

### Summary of the invention

It is an object of the present invention to provide a method for screening an age-related candidate substance or an age-related candidate gene comprising the steps of:
a) expressing at least one polypeptide comprising a nuclear localization signal and a farnesylation site using means for genetic expression;
b) applying at least one candidate substance or at least one candidate gene to the means; and
c) measuring at least one aging parameter from the means,
wherein a modification of the aging parameter indicates an age-related substance or an age-related gene.

Furthermore, the invention concerns an assay for screening age-related candidate substances or age-related candidate genes comprising at least one means for expressing at least one polypeptide and a system for measuring at least one aging parameter, wherein the means comprises a nuclear localization signal and a farnesylation site.

In addition, the present invention is directed to a nucleic acid encoding a polypeptide comprising a nuclear localization signal, a farnesylation site and a conditional expression sequence.

The invention relates also to the use of the assay for screening at least one age-related candidate substance or at least one candidate age-related gene.

### Brief description of drawings

Figure 1A shows the morphology of longitudinal muscle nuclei in aging *Drosophila* flies by fluorescent antibody staining. Muscle nuclei of wild-type flies (wt), RU 486 induced upstream activated sequence (UAS) lamin B/Gene Switch-myosin heavy chain (Iamin B/GS-MHC) longitudinal muscle nuclei, and UASkuk/GS-MHC longitudinal muscle nuclei at indicated ages were stained by fluorescent kugelkern protein (Kuk) antibody.
In Figure 1B perimeters of wild type (upper panel), UASIamin B/GS-MHC (middle panel), or UASkuk/GS MHC (lower panel) flies longitudinal muscle nuclei at indicated ages are plotted (grey bars: non-induced control flies, black bars: RU 486 induced flies).
Figures 1C-K show transmission-electron microscopy images of muscle cells of wt, kuk, UASkuk/GS-MHC, UASIamin B/GS-MHC and UASkuk/GS-MHC induced by RU 486.
Figure 2A shows the overexpression of protein lamin B or protein Kuk that may affect *Drosophila* life span with 0, 4 or 6 genomic copies of kuk in cellularizing embryos.
Figure 2B shows that the mean life span of flies is reduced if they contain 4 or 6 genomic copies of kuk.
Figure 2C displays survival experiments of flies overexpressing lamin or kuk in specific tissues via GS where expression was induced by RU 486 added to the food of adult flies (solid lines and circles).
Figure 2D depicts age-specific mortality rates of induced and control flies plotted on a log scale versus time.
Figure 3A shows DNA damage in adult flies by H2A.X staining and gene silencing by a reporter of position effect variegation in 1 week or 5 weeks old wt longitudinal muscle nuclei.
In Figure 4 overexpression of IaminBΔN or kuk inducing DNA damage and changing levels of nuclear proteins in cultured mouse fibroblast cells are demonstrated.
In Figure 5A and B the tissue specific induction of lamin B and kuk expression in 10 days old males is quantified, respectively.

### Detailed description of the invention

The present invention is directed to a method for screening an age-related candidate substance or an age-related candidate gene comprising the steps of:
a) expressing at least one polypeptide comprising a nuclear localization signal and a farnesylation site using means for genetic expression;
b) applying at least one candidate substance or at least one candidate gene to the means; and
c) measuring at least one aging parameter from the means,
wherein a modification of the aging parameter indicates an age-related substance or an age-related gene.

The term "screening" as used herein means detecting age-related candidate substances from a pool of unknown substances and compounds to identify them as pharmacologically active substances, which modify aging. The term "screening" as used herein also means detecting age-related candidate genes from a pool of genes to identify them as genes, which regulate aging. The pool comprises at least one up to a plurality of substances or genes. High-throughput screening of a large pool of potential substances or genes is also enclosed, because it is an efficient method for drug discovery, drug design and gene identification.

The term "age-related" as used herein refers to substances or genes that are associated with normal and pathological aging. Age-related substances impair symptoms and causes of aging.

The term "polypeptide" as used herein refers to chains of amino acids regardless of length and conformation. Therefore, the term "polypeptide" comprises oligomers, polymers as well as proteins.

The term "nuclear localization signal" (NLS) as used herein refers to a natural or artificial amino acid sequence, which acts like a tag used to target a compound, e.g. a newly synthesized protein to the cell nucleus through the nuclear pore complex. The protein with the NLS is directed into the nucleus via its recognition by cytosolic nuclear transport receptors. Different types of NLS are known, but one common feature is a few short sequences of positively charged lysines or arginines. Different nuclear localized proteins may share the same NLS.

The term "farnesylation site" as used herein refers to an amino acid sequence with a farnesyl group that is posttranslationally added by a prenyltransferase, e.g. a farnesyltransferase. The farnesyl group is a 15-carbon isoprenoid, like farnesol and geranylgeraniol, optionally bearing a four amino acid sequence, preferably a CaaX motif at the carboxyl terminus of a protein. Targets of farnesyltransferase include members of the Ras superfamily of small GTP-binding proteins critical to cell cycle progression.

The term "means for genetic expression" as used herein refers to any kind of natural and artificial vehicles of genetic information that is transformed into a functional gene product, like a protein or RNA. The means are in vivo, ex vivo and in vitro systems including their different developmental stages. It may concern wild type means or genetically manipulated means.

The term "applying" as used herein refers to any kind of contacting the candidate substance with the means. The term "applying" as used herein refers also to inserting the gene to be investigated into an expression means that is the same or different to the one expressing at least one polypeptide comprising a nuclear localization signal and a farnesylation site. Further, the term "applying" also refers to the expression of the applied gene. "Applying" includes methods like injection, transposition, transduction, transfection, conjugation, transformation, splicing and activation. "Applying" includes also uncaging, releasing, blending, incubating, adding, dispensing, and administering.

The gene or substance can be administered locally, e.g. topically, or systemically, e.g. orally. The candidate substances may be admixed with food or drinking. Genes and substances can be added to the means into any kind of laboratory vessel, like vials, tubes, or 96-well plates. For high-throughput screening an array can be used to contact a plurality of candidate substances or genes with the means.

The term "aging parameter" as used herein refers to parameters that display the causes and symptoms of aging. It may concern molecular, cellular and physiological parameters, or the like.

Aging parameters can be measured by any measuring system, like biochemical, diagnostic, histological, and physiological methods, e.g. histological stainings and behavioral tests. The term "measuring" also refers to reading out and analyzing data by calculating or statistical methods. The measuring system includes quantitative methods, qualitative methods and mixtures of both.

The invention is based on the finding that polypeptides comprising a nuclear localization signal and a farnesylation site can induce aberrant nuclear shapes with nuclear envelope foldings, lobulations and extra membrane growth. Scientists are still working on the question how changes in nuclear morphology relate to cellular aging and aging of organisms. It might be possible that irregular shaped nuclei are simply a consequence of aging in cells. In cultured HGPS cells, it has been shown that loss of H3K27me3, a marker for facultative heterochromatin, takes place before morphological changes of nuclei become visible (Shumaker et al., 2006) arguing that nuclear shape changes are a consequence of aging. However, the data shown by the inventors demonstrate that an abnormal nuclear architecture is able to promote the aging process itself. Nuclear shape changes were induced in a IaminΔ50 independent way, and their effect on age-related phenotypes on the level of cells and organisms were tested. Indeed, farnesylated lamina proteins, like lamin B or Kuk, cause aberrant nuclear shapes that shorten the life span of flies. Therefore, functional evidence is provided that kuk contributes to the regulation of heterochromatin formation in the fly. Moreover, even a truncated lamin B variant that only consists of the nuclear localization signal and the C-terminal farnesylation site as well as the *Drosophila* specific Kuk are able to induce age-related cellular defects. These new findings support that changes in the nuclear lamina may cause an aging process.

By the expression of polypeptides comprising a nuclear localization signal and a farnesylation site, age-dependent nuclear changes can be induced. As a consequence of the nuclear changes aging-like phenotypes are provoked, wherein the nuclear changes are in a causal relationship to the aging-like phenotypes. Therefore, the invention provides a new method and an assay to screen and investigate candidate substances and genes for their ability to affect aging. Thus, the invention provides a tool to discover and develop therapies and drugs that delay normal aging or remedy age-related diseases, like degenerative diseases or accelerated aging diseases. In addition, it provides a tool to identify genes that play a role in aging or age-related diseases.

The expression of polypeptides according to the invention provides a homogeneous system that affords reproducible and comparable results. In a preferred embodiment the polypeptides are expressed in immortalized cell cultures that provide a stable system displaying aging phenotypes by induction without tedious culturing.

The invention can be performed on the level of molecules, cells, organelles, or organisms. Therefore, a large variety of aging parameters can be investigated, and the output in terms of data, useful drugs and therapies or identified genes is broadly applicable.

In preferred embodiment the aging parameter is selected from the group consisting of DNA stability and damage, nuclear morphology, chromatin formation, histone modification, gene expression, behavioral pattern of the means, organelle modification, and organelle distribution. The term "DNA stability and damage" includes oxidation, hydrolysis, alkylation and mismatch of bases, but also mutations, like point mutations, insertions, and deletions, and large scale aberrations in chromosomes, like inversions, amplifications and translocations. The term "nuclear morphology" refers to the stability and shape of the nucleus that is altered e.g. by lobulation or envelope foldings. It also refers to nucleoplasm, nuclear membrane, nuclear pores and nuclear proteins. The term "chromatin formation" refers to the DNA/protein structure of chromosomes that can be modified e.g. by condensation, inclusions, methylation, phosphorylation and acetylation, like posttranslational histone dimethylation. The term "gene expression" includes activating and silencing of gene transcription, translation and intermediate modifications, like splicing. The term "behavioral pattern" includes motor and social behavior, cognition, behavior due to learning and memory as well as behavior of structures, molecules and cells. Analysis of behavioral patterns is conducted by experimental behavioral tests.

In a another preferred embodiment the means is selected from the group consisting of prokaryotes; animals; insects, preferably *Drosophila melanogaster,* nematodes, preferably *Caenorhabditis elegans;* plants; fungi, preferably *Saccharomyces cerevisiae;* protists; transgenic vectors, like viral, bacterial and plasmid vectors, artificial yeast chromosomes; viruses; cells, preferably insect amphibian or mammalian cells. *Xenopus laevis* oocytes, NIH3T3 and HeLa cells are most preferred. *Drosophila melanogaster* is a preferred means, because these flies can be kept economically in huge amounts, and have a fast alteration of generation. In addition, they show distinct behavioral patterns and can be easily genetically manipulated.

In a preferred embodiment the polypeptide is selected from the group consisting of kugelkern (Kuk), A type lamin and B type lamin, preferably IaminBΔN.

Kuk contains a putative coiled-coil domain in the N-terminal half of the protein, a NLS, and a C-terminal CxxM-motif for targeting Kuk to the inner nuclear membrane. Kuk functions in nuclear elongation. Recent findings suggest that Kuk is a rate-determining factor for nuclear size increase (Brandt et al., 2006). Without being bound to any theory, the inventors believe that association of farnesylated Kuk with the inner nuclear membrane induces expansion of the nuclear surface area, allowing nuclear growth.

Lamins are intermediate filament proteins regulating structural function and transcription in the cell's nucleus. These proteins localize to two regions of the nuclear compartment, the nuclear lamina and throughout the nucleoplasm. They are essential proteins that provide mechanical stability to a nuclear envelope. In addition, they influence DNA replication, and organize the chromatin bordering the inner nuclear membrane by binding to chromatin and inner nuclear membrane. Lamins are also required for RNA-polymerase II-dependent transcription and are involved in the localization of transcriptional factors. There are indications that lamins affect nuclear growth after the re-formation of the nuclear envelope.

Lamin contains an alpha-helical rod domain centered between a non-helical N-terminal head domain and a C-terminal globular tail domain. The C-terminal domain contains a NLS, an Ig-fold, like domain, and in most, but not all cases a C-terminal CaaX box that is isoprenylated and carboxymethylated. These modifications confer a high hydrophobicity to the C-terminus and are essential for targeting to the inner nuclear membrane.

There are several types of lamin, like A type lamins, e.g. lamin A and C, and B type lamins, e.g. lamin B1 and B2, which differ in their length and isoelectric point. In human cells three differentially regulated genes are known, LMNB1, LMNB2 and LMNA. B type lamins, like B1 and B2, are present in every cell and are expressed from the LMNB1 and LMNB2 genes on 5q23 and 19q13, respectively.

Lamin BΔN is a lamin B variant truncated at the N-terminus. Preferably it comprises a nuclear localization signal and a C-terminal farnesylation site.

Age-dependent morphological alterations can be induced ahead of time by overexpressing the polypeptides according to the invention. In a preferred embodiment the lifespan of the means is reduced, preferably up to 60%, more preferably up to 70%.

In another preferred embodiment an age-related phenotype of the means appears within 48 h, preferably within 24 h. These advantages help saving costs, time and therefore labor input.

In another preferred embodiment the polypeptide is conditionally expressed, preferably by a gene switch sequence. The term "conditionally expressed" as used herein refers to a restricted gene expression that occurs only under distinct conditions. The expression can be spatially or temporally restricted. One method to express transgenes in a spatially restricted manner is a light-induced activation of genes, like activation of a heat-shock protein by a laser micro beam or light activation of caged molecules. Another method is a bipartite expression system based on the yeast GAL4 protein and its upstream activating sequence. There is a wide variety of driver lines with defined expression patterns generated e.g. by using cloned promoter fragments or enhancer detector screens. Temporal control transgene expression can be accomplished by using e.g. a heat-shock promoter or tetracycline-dependent "tet-off" or "tet-on" transactivation systems.

Spatially and temporally controlled expression of the polypeptide is a preferred embodiment. This can be accomplished by a fusion protein of GAL4 with receptors of inducing ligands, like hormones, e.g. estrogens or progesterone. It can also be accomplished by a gene switch. The gene switch is based on a chimeric gene that encodes the GAL4 DNA binding domain, a ligand binding receptor-domain, and an activation domain. In the presence of a ligand the chimeric molecule binds to the UAS and provides for ligand inducible activation of downstream target genes. Examples of a spatially and temporally controlled expression by using tissue specific promoters and a systemic application of the ligand is the RU486 induction of UASlamin/GS-MHC, UASlamin/GS-Sl 106, UASlamin/GS-elav, UASkuk/GS-MHC, UASkuk/GS-S1 106 or UASkuk/GS-elav.

The present invention is also directed to an assay for screening age-related candidate substances or age-related candidate genes comprising at least one means for expressing at least one polypeptide and a system for measuring at least one aging parameter, wherein the means comprises a nuclear localization signal and a farnesylation site.

In a preferred embodiment the means comprises a conditional expression sequence, preferably a gene switch sequence.

In a further preferred embodiment of the assay the polypeptide is selected from the group consisting of Kuk, A type lamin and B type lamin, preferably IaminBΔN.

In another preferred embodiment the assay further comprises at least one candidate substance or at least one candidate gene.

The present invention also relates to a nucleic acid encoding a polypeptide comprising a nuclear localization signal, a farnesylation site and a conditional expression sequence.

In a preferred embodiment the polypeptide is selected from the group consisting of kugelkern (Kuk), A type lamin and B type lamin, preferably IaminBΔN.

Loss-of-function mutations in lamin genes lead to laminopathies, like Emery-Dreifuss muscular dystrophy, familial partial lipodystrophy, limb girdle muscular dystrophy, dilated cardiomyopathy, Charcot-Marie-Tooth disease, and gain-of-function laminopathies like HGPS. Therefore, nucleic acids according to the invention encoding lamin or Kuk with different expression patterns or mutations can serve as models for these diseases or other age-related or degenerative diseases.

It is possible to use these mutants for studying aging processes, but it is difficult to distinguish defects caused throughout development and defects directly related to aging. Therefore, in a preferred embodiment the conditional expression sequence is a gene switch sequence, preferably selected from the group consisting of the gene switch-myosin heavy chain sequence (GS-MHC), the gene switch-embryonic lethal abnormal vision sequence (GS-elav) and the gene switch-S1 106 sequence.

GS-MHC is a muscle specific GS driver that uses the promoter of the MHC gene with a panmuscular distribution. The GS-elav is a GS driver specific for neuronal tissue that uses the promoter from the elav gene with a panneural distribution. The GS-S1 106 drives gene expression in the fat body of insects and white adipose tissue. A reporter gene expression can be regulated by the dosage of the activating ligand RU486.

Overexpression of lamin B, or C, human LMNA, or IaminΔ50 in *Drosophila* using the UAS/GAL system leads to a severely affected viability, resulting in low eclosing rates and early mortality. To approximate the situation in HGPS where IaminΔ50 acts dominantly, overexpression of the gene variants should be in a time and tissue specific manner. The nucleic acid comprising a gene switch system according to the invention allows to minimize the problem of genetic background as well as to circumvent defects which may arise during development. Therefore, it is possible to analyze effects of candidate substances on aging based on conditional lamin B or kuk expression in adult flies.

Further, the invention relates to the use of the assay according to the invention for screening at least one age-related candidate substance or at least one age-related candidate gene.

In a preferred embodiment the substance is a pharmaceutically active substance for therapeutic and/or prophylactic treatment of age-related disorders like Alzheimer's disease, cancer, Parkinson's disease, HGPS, and other laminopathies.

### Examples

### 1. Methods

### 1.1 Cell culture

NIH3T3 cells were cultured in DMEM (Invitrogen, GIBCO) supplemented with 10% FBS and 2 mM L-glutamine at 37°C. NIH3T3 cells were plated in 6-wells containing cover slips and were transiently transfected with Effectene (Qiagen), when they reached a confluence of 25 - 30% with pCS2HAkuk (Brandt et al., 2006) or pCS2XlaminBΔNGFP (Prüfert et al., 2004) (2 µg construct/6-well). They were cultivated for 48 h for heterochromatin protein 1 (HP1) and Tri-Me-H3K9 staining or for 72 h for H2A.X staining. After washing in phosphate buffered saline (PBS), cells were fixed with 2% formaldehyde in PBS containing 0.2% Tween and 0.5% NP-40 for 20 min at room temperature (RT). After washing in PBS cells were permeabilized in PBS with 0.5% Triton X-100 plus 0.5% Saponin (Sigma) for 10 min.

### 1.2 Immunohistochemistry

Adult male flies were anaesthetized. The head, abdomen, legs, and wings were cut off. The thorax was transferred to ice cold Schneider cell medium, where it was split into half and subsequently fixed with 8% formaldehyde in PBS containing 0.2% Tween and 0.5% NP-40 for 40 min at RT. After washing in PBS the thoraces were permeabilized in PBS with 0.5% Triton X-100 plus 0.5% Saponin (Sigma) for 1 to 5 days at 4°. Before staining, the fixed thoraces were manipulated that only one end of the muscles stuck to the cuticle and the other end was free floating and better accessible to the staining reagents. Consecutively, muscles were blocked in PBT with 5% BSA and stained in PBT containing primary antibodies, fluorescent secondary antibodies (4 µg/ml, Alexa, Molecular Probes) or DAPI and mounted in Aquapolymount (Polyscience). Antibodies used were A414 (Sigma, 1 µg/ml), *Drosophila* HP1 (DSHB, C1A9, H2A.X (Chemicon, 1:5000), mouse HP1 (Chemicon, 1:2500), PCS2HAkuk (0.2 µg/ml), Tri-Me-H3K9 (upstate, 0.2 µg/ml), 0.1 µg/ml), *Drosophila* lamin B (LaminDmO, 0.1 µg/ml), human IaminA/C (Santa Cruz Biotechnology, 0.2 µg/ml) and HA (Babco, 1:2000).

### 1.3 Microscopy

Digital fluorescent images were either taken with a confocal microscope (Leica) or with a fluorescent microscope connected to a Progress camera and processed with Photoshop (Adobe). Measurements were performed with ImageJ by the perimeter tool to measure the nuclear perimeter, by the mean grey value tool to measure the relative fluorescent intensity and by the maximum intensity tool. For measuring relative fluorescent intensity of HP1 and Tri-Me-H3K9 stainings, three independent experiments were analyzed by the fluorescence microscope. For each experiment the maximum intensity was set as 100%. The number of H2AX foci per nucleus might be underestimated since only those foci that were in the focus plane of the picture were counted. (Mann-Whitney U-Test, http://faculty.vassar.edu/lowry/VassarStats.html). For measurements of the muscle perimeters, confocal image z-stacks of the longitudinal muscles with a slice distance of approx. 1 µm were acquired and assembled in ImageJ with the z-stack/SUM function. For each time point three individual flies were analyzed. Statistical analysis was performed with Prism GraphPad software, (One- or Two-Way-ANOVA followed by Bonferronis post tests).

### 1.4 Electron microscopy

Thoraces were fixed and processed for electron microscopy as described in Fryberg et al. (1990). In contrast to Fryberg et al., the tannic acid from the 3% glutaraldehyde solution was omitted. Ultra thin sections were inspected with a Zeiss EM900 and a Zeiss EM10 electron microscope (Oberkochen, Germany). Negatives were digitalized by scanning and processed with Adobe Photoshop.

### 1.5 Drosophila strains

Gene switch-Gal4 flies and w (superscript: m4h) flies were provided and UASIamin B flies were prepared as described in Guillemin et al. (2001). For pUASkuk the cDNA from LD09231 was cloned as Notl-Apal/blunt fragment into the Notl-Xbal/blunt sites of pUASp. To obtain heat-shock inducible HS-kuk full length or mutant HSkukCSflies, kuk CDS with parts of the 3'-UTR as Ncol-Smal PCR fragments of CS-kuk or CS-kukCS into QEH₁₀ZZ was cloned. Subsequently, HS-kuk or mutant HSkukCS was cloned as EcoRI-Smal fragments from QEH₁₀ZZ-kuk or -kukCS into EcoRI-Stul of CasperHS. Transgenic flies were made by P-element mediated transformation. The genomic rescue constructs 4x and 6x kuk are located on the second and third chromosome. 0x kuk consists of a deletion of kuk and CG5169 together with a rescue construct of CG5169 over Df(3R)Ex6176. To induce conditional expression of the full-length kuk or the non farnesylated CaaX-mutant kuk (kukCS) in wt or in kuk deficient embryos (Δkuk), transgenic embryos containing the respective heat-shock constructs were heat-shocked for 45 min. After 45 min recovery the embryos were fixed and stained with Kuk or lamin B antibodies.

### 1.6 Life span assays

All flies were raised and kept in a humidified, temperature-controlled incubator with 12 h on/off light cycle at 25°C in vials containing standard cornmeal medium (2.5% yeast, 2.18% treacle, 1% soy meal, 8% cornmeal, 8% malt, 1.25 % propionic acid). In the case of UASlamin B/GS-MHC, flies were raised at 18°C. Flies were collected under short anesthesia. Each demography cage was initiated with at least 150 newly eclosed males. The number of deceased flies was recorded every 2 to 3 days, when flies were transferred to fresh food plates. For induction with the GS system, RU 486 (Sigma) was added directly to the food to a final concentration of 200 µM. The data across 3 to 5 replicate demography cages per treatment and genotype were combined. Both Statview 5.0 and Prism GraphPad software were used to analyze survival data by a log-rank test and mortality curves by linear regression.

### 1.7 Negative geotaxis assays

Negative geotaxis was assessed as previously described in Gargano et al. (2005). Negative geotaxis behavior was recorded as the net distance in cm climbed by individual males in a vertical plastic tube during a 10 second test period that began immediately after being tapped to the bottom of the tube. For each genotype and treatment 25 males were tested per week. The mean distance climbed by the control flies in the first week was set as 100% negative geotaxis. One to three replicative experiments were performed and the data were analyzed using Prism 3.0 (GraphPad Software, San Diego, CA, USA).

### 1.8 Eye pigment measurement

10 newly eclosed males were aged for 5 days at 25°C, decapitated and immediately shock-frozen in liquid nitrogen. For extraction of the eye pigments, fly heads were homogenized in 0.1 M glycine/HCl (pH 2) containing 30% ethanol and kept in the dark for 24 h at room temperature. After clarification by several brief centrifugations, the absorption of the pigments was measured at 480 nm. Five independent extractions were performed for each genotype. VassarStat statistical software was used for Mann-Whitney tests.

### 1.9 Biochemistry

Proteins were analyzed by SDS polyacrylamid gel electrophoresis (SDS-PAGE) according to standard protocols. For western-blotting proteins separated by SDS-PAGE were transferred by semi-dry blotting to a nitrocellulose membrane (Protran, Schleicher and Schuell) and stained by Ponceau Red. The blots were developed with IgG coupled with peroxidase and chemiluminescence (ECLplus, Amersham). Following antibody concentrations were used: lamin B, polyclonal: 1:1000, Kuk: rabbit, 0.1 µg/ml.

### 2. Results

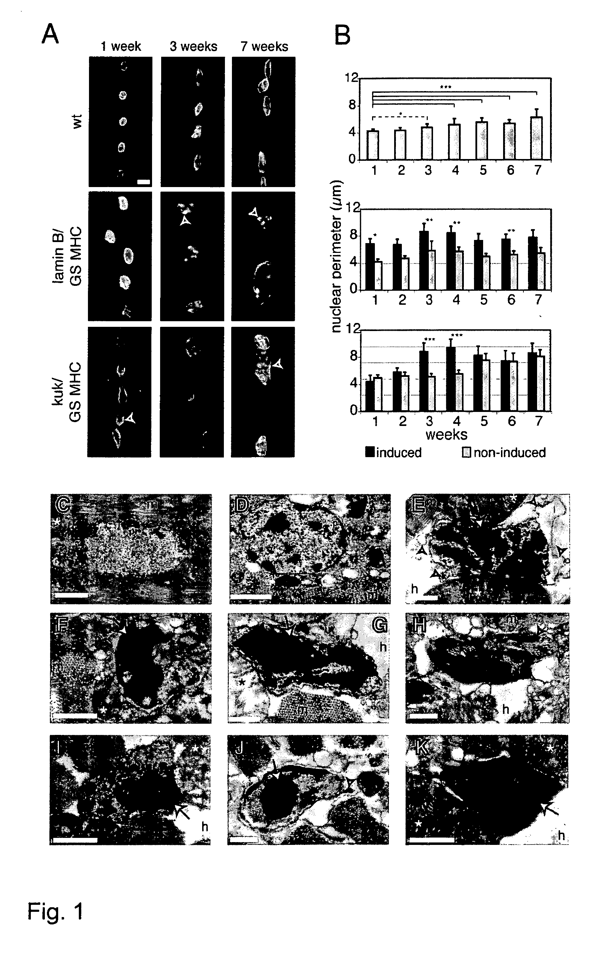

Figure 1A shows the morphology of longitudinal muscle nuclei in aging *Drosophila* flies by fluorescent antibody staining. Muscle nuclei of wt flies, UAS lamin B/GS-MHC, and UASkuk/GS-MHC longitudinal muscle nuclei at indicated ages were stained by fluorescent Kuk antibody (scale bar: 1 µm) (Figure 1A). Z-stacks were fused with the maximum intensity tool in ImageJ to show several nuclei.

In Figure 1B perimeters of wild type (upper panel), UASlamin B/GS-MHC (middle panel), or UASkuk/GS MHC (lower panel) flies longitudinal muscle nuclei at indicated ages are plotted (grey bars: non-induced control flies, black bars: RU 486 induced flies). In wt flies number (n) is between 45 and 95 nuclei per time-point, in UASlamin B/GS-MHC flies n is between 41 and 135 nuclei per time-point and in UASkuk/GS-MHC flies n is between 37 and 104 nuclei per time-point. Mean values are depicted with ± standard error of means (s.e.m.). For wt flies a One-way ANOVA and for UASlamin B/GS-MHC or UASkuk/GS-MHC flies a Two-way ANOVA were followed by Bonferronis Multiple Comparison Test, * p < 0,05, ** p < 0,01, *** p < 0,0001.

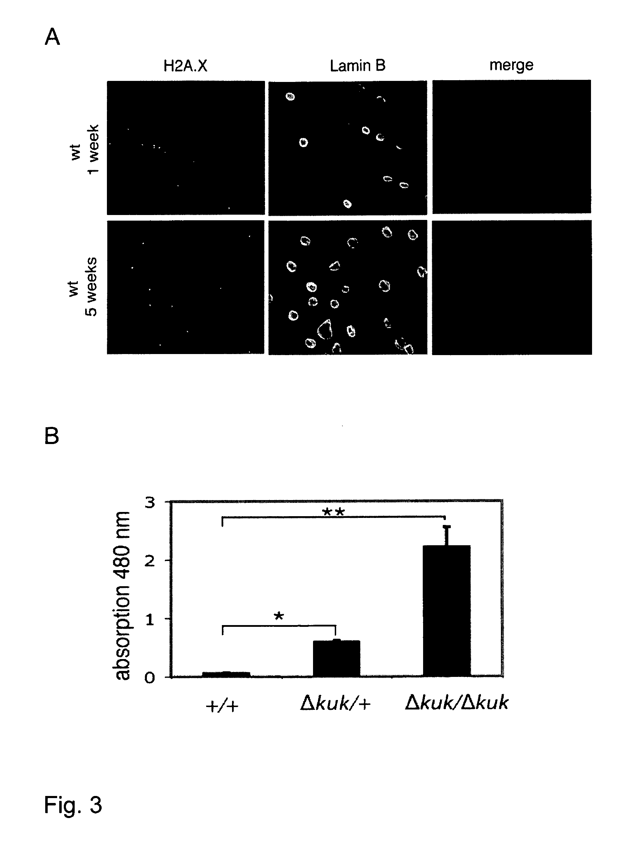

Nuclear architecture in adult *Drosophila* longitudinal muscle cells shows progressive and stochastic alteration as fly ages (Figure 1). Most muscle nuclei in young animals were round and possessed a smooth surface. Over time, the nuclei increased significantly in size and assumed an uneven wrinkled shape (Figure 1A, B). When lamin B or kuk were overexpressed in adult muscle cells, a significant increase in nuclear perimeter, wrinkled, lobulated nuclei, and accumulations of Kuk (Figure 1A, B) or lamin B staining (Figure 3A) was observed at the nuclear envelope.

Figures 1C-K show transmission-electron microscopy images of muscle cells with the following legend: (C) wt, 3 days old; (D) wt, 35 days old; (E) wt, 60 days old; (F) 6 genomic copies of kuk (6x kuk), 3 days old; (G) 6x kuk, 30 days old; (H) 6x kuk, 36 days old; (I) UASkuk/GS-MHC induced with RU 486, 3 days old; (J) UASlamin B/GS-MHC induced by RU 486, 1 week old; (K) UASkuk/GS-MHC induced by RU 486, 31 days old; muscle fibers (m); arrowheads mark nuclear protrusions; (h) organelle free holes in the tissue; arrows mark electron dense areas containing probably highly condensed chromatin; peripheral heterochromatin (p); lumen between inner and outer nuclear membrane (I); mitochondria (*); scale bar: 0.5 µm.

Muscle nuclei of Figures 1C - K showed an age-dependent loss of peripheral heterochromatin and a strong increase of dark inclusions, which may contain highly condensed chromatin. Moreover, there was a separation of inner and outer nuclear membrane. In old nuclei, high numbers of ring-like structures with less than 50 nm in diameter in the nucleoplasm were found. These structures were too small to present a complete nuclear pore. The ring-like structures were not marked by the nuclear pore-marker AB414, lamin B, or Kuk antibodies in immuno-electron microscopy. No trilaminar unit-membrane structure was visible in the rings, which argues against vesicles. Although actin/myosin arrays of the muscle fibers seemed to be intact in old flies, the number of degenerated mitochondria increased and organelle free zones were found. In lamin B or kuk overexpressing flies, loss of peripheral heterochromatin, separation of inner and outer nuclear membrane, accumulation of ring-like structures, and a strong increase of dark material in the nucleoplasm were observed even in 1 week old flies. In tissues, which were not overexpressing lamin B or kuk, nuclear size or shape did not alter. In conclusion, age-dependent morphological changes of the nucleus in *Drosophila* flies were described that can be induced ahead of time by overexpressing lamin B or kuk.

The inventors surprisingly found that a farnesylation motif is relevant for kuk activity. The kukCS mutant allele, which lacks its farnesylation site in kuk deficient mutant fly embryos by heat-shock, was expressed, and its localization and action towards nuclear morphology were characterized. Predominantly nucleoplasmatic localizations of KukCS protein, with only few small dots localized to the nuclear envelope were found. Interestingly, when the non-farnesylated KukCS protein in wt flies was expressed, there was a clear localization to the nuclear envelope. Moreover, in the wt background, the mutated protein was able to induce lobulation of nuclear membrane, albeit weaker than that induced by normally farnesylated Kuk. The finding that KukCS cannot localize to the nuclear envelope in the kuk deficient background as well as its inability to induce nuclear lobulation argues that the farnesyl residue is indeed required for Kuk localization and function.

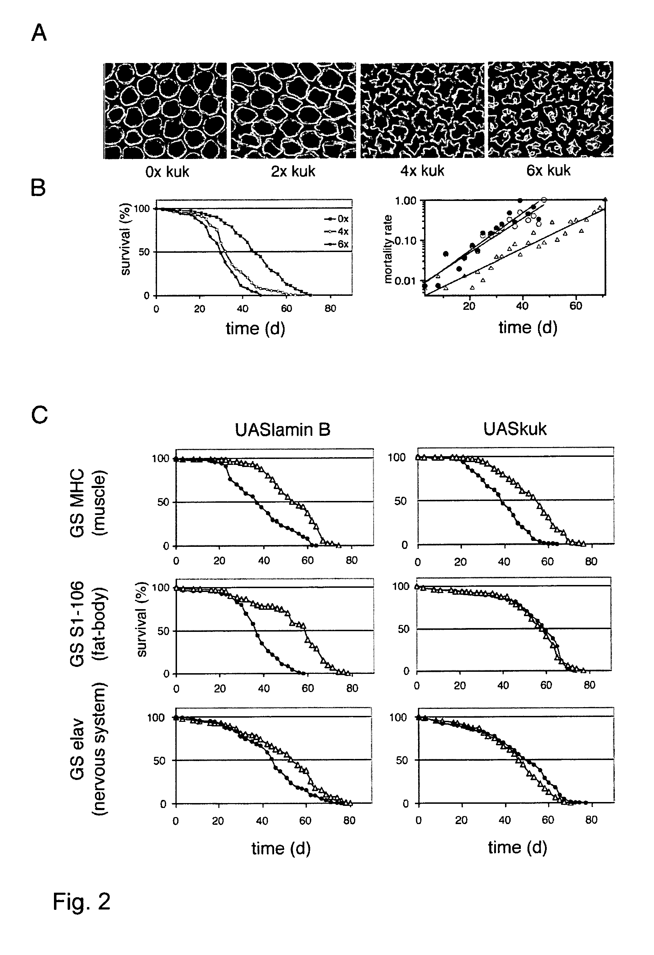

Figure 2A shows the overexpression of proteins lamin B or Kuk that shortens *Drosophila* life span with 0, 4 or 6 genomic copies of kuk in cellularizing embryos. Nuclei were stained with lamin B antibodies, and apical sides of nuclei in early cycle 14 are shown. In the absence of kuk, the nuclei are round and smooth. With increasing amounts of kuk, the nuclei show deep infoldings of the nuclear envelope.

Figure 2B shows that the mean life span of flies is reduced if they contain 4 or 6 genomic copies of kuk.

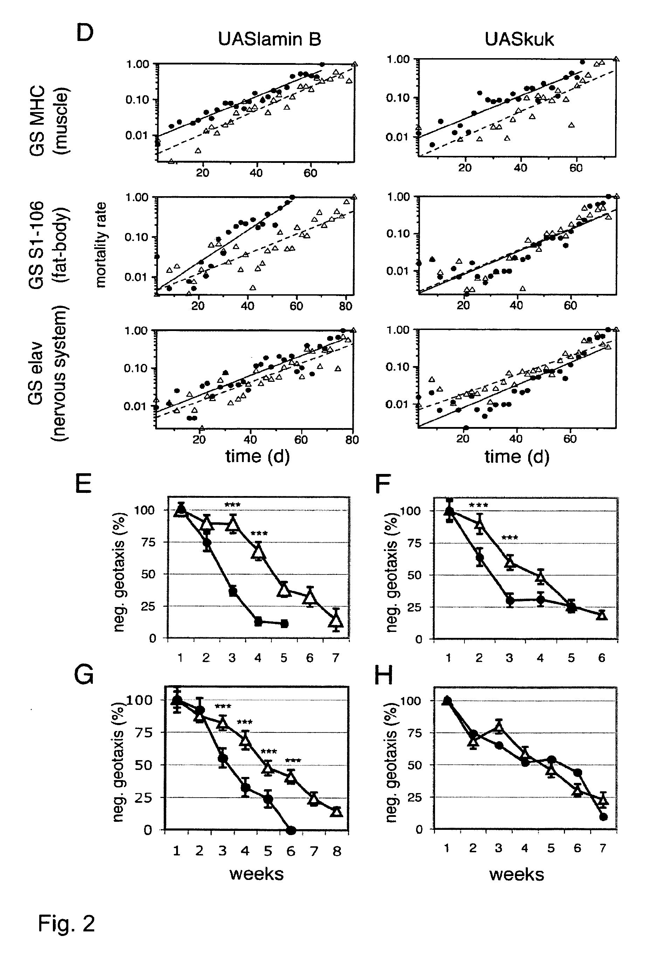

Figure 2C displays survival experiments of flies overexpressing lamin or kuk in specific tissues via GS where expression was induced by RU 486 added to the food of adult flies (solid lines and circles). Control flies had the same genotype, but were not fed with RU 486 (dashed lines, open triangles). Log-rank tests compare induced and control flies. The legend of Figure 2 is as follows: UASlamin/GS-MHC males: + RU 486, n = 120, 4 experiments; - RU 486, n = 214, 3 experiments; log-rank test: p < 0.001. UASlamin/GS-S1 106, expressed in fat bodies: + RU 486, n = 454, 3 experiments; - RU 486, n = 353, 3 experiments; log-rank test: p < 0.001. UASlamin/GS-embryonic lethal abnormal vision (elav): + RU 486, n = 441, 3 experiments; - RU 486, n = 421, 3 experiments; log-rank test: p < 0.001; UASkuk/GS-MHC: + RU 486, n = 558, 5 experiments; - RU 486, n = 811, 7 experiments; log-rank test: p < 0.001. UASkuk/GS-S1 106: + RU 486, n = 454, 3 experiments; - RU 486, n = 353, 3 experiments; log-rank test: p = 0.0181. UASkuk/GS-elav: + RU486, n = 441, 3 experiments; - RU 486, n = 421, 3 experiments; log-rank test: p < 0.001.

Figure 2D depicts age-specific mortality rates of induced and control flies plotted on a log scale versus time, fitted by Gompertz model (In(mx) = In(m0) + ax), where mx = mortality rate at age x, m0 = baseline mortality (intercept as In(m0)) and ax = change of mortality with age (slope of the trajectory). The slope of the mortality trajectory only differs significantly between induced and control UASlamin B/GS-S1 06 flies (p = 0.005). Significantly different Y-intercepts between induced and control groups are found in UASlamin/GS-MHC (p = 0.0002), UASlamin/GS-elav (p = 0.01), or UASkuk/GS-MHC flies (p = 0.04).

Induced expression of farnesylated proteins affecting lamina structure also provoked aging-like phenotypes (Figure 2A). Flies containing 0, 4, or 6 genomic copies of kuk for their longevity were tested. An increase in the dosage of kuk or lamin shortened the life span of the adult flies (Figure 2B-D). To minimize the problem of genetic background as well as defects which may arise during development, tissue specific drivers inducible with RU 486 were used, wherein the difference between the experimental and control condition is the presence or absence of the inducing agent in the food of the adult flies called GS system. Conditional expression of lamin B or kuk in the adult muscle cells via the muscle specific gene switch driver GS-MHC decreased the life span compared to un-induced control flies of the same genotype. UASkuk/GS-MHC flies showed 60% and UASlamin B/GS-MHC flies showed 54% reduction of mean life span. Conditional expression of lamin B in the abdominal fat body, the fly equivalent of the liver and white adipose tissue via the GS-S1-106 driver reduced the mean life span to 46% of un-induced control flies. Expression in the nervous system in UASlamin B/GS-elav flies from the onset of adulthood decreased the mean life span not significantly (90%). The increase of mortality rates of RU 486 induced kuk/GS-MHC, lamin B/GS-MHC, and lamin B/GS-elav flies were similar to non-induced flies, but their onset differed. An increase in the initial mortality may argue for a deleterious effect of lamin B or kuk overexpression. In lamin B/GS-S1 106 induced flies, a change in the demographic rate of aging was observed.

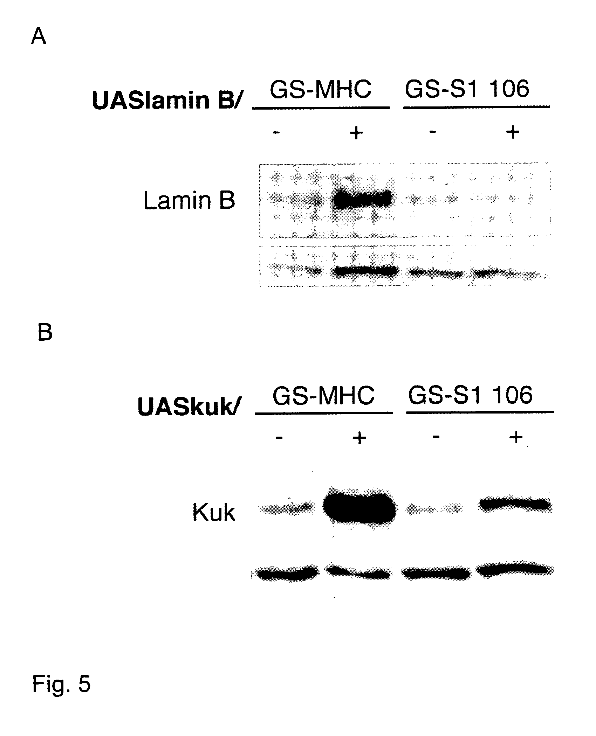

The variations between UASkuk and UASlamin B in the tissue specific driver lines may be due to different expression levels or may reflect tissue specific functions. To test whether the life span data correlate with the morphology of the nuclei in the lamin B or kuk expressing tissues, nuclear size in fat body cells or in the optic lamina of three weeks old RU 486 induced flies vs. control flies was examined. The nuclear size in lamin B expressing fat body nuclei compared to control nuclei increased, but nuclear size in kuk expressing cells did not change. In lamin B expressing neuronal tissue nuclear size increased. In kuk expressing brains only few neurons showed changes in nuclear size. Figure 5 quantifies the tissue specific induction of lamin B and kuk expression in 10 days old males. The legend of Figure 5 is as follows: (+) means RU 486 induced expression, (-) means un-induced flies. The strongest induction of Kuk is observed with the GS-MHC driver.

Thus, the nuclear morphology of lamin B or kuk expressing cells correlate with the tissue specific effects observed in life span experiments. To better analyze the system of the invention, the levels of lamin B or kuk expression in induced flies were investigated and a moderate induction of lamin B or kuk expression in the different tissues was displayed (Figure 5). Between the different driver lines, the GS-MHC driver showed the strongest increase in UASkuk or UASlamin B expression compared to the lower induction by GS-S1 106 or the GS-elav line. The more prominent induction of kuk or lamin B expression via the GS-MHC driver correlates with a strong effect on life span in these lines. Induction driven by the GS-S1 106 or the GS-elav driver seems to be less pronounced and correlates with a more differential effect on life span. These flies appear to be more sensitive to lamin B than to kuk overexpression. In conclusion, these data argue for a correlation of induction levels with phenotypic strength and not for a tissue specific effect of lamin B or kuk expression.

Negative geotaxis, an innate escape response during which flies climb the wall of a cylinder after being tapped to its bottom, is a well characterized behavior that senesces in *Drosophila.* In Figure 2E - H negative geotaxis of RU 486 induced UASkuk/GS-MHC (Figure 2E; 3 replicate experiments), UASlamin B/GS-MHC (Figure 2F; 3 replicate experiments), UASlamin B/GS-S1 106 (Figure 2G; 3 replicate experiments), and UASkuk/GS-elav (Figure 2H; 1 experiment) expressing flies (closed circle) are compared with un-induced control flies (open triangle). The mean distance climbed by the control flies in the first week is set as 100% negative geotaxis. One-way ANOVA was followed by Bonferronis Multiple Comparison Test, *** = p< 0,001. Statistical tests are two-tailed, mean values are shown with error bars ± s.e.m.. An early decline in the climbing ability in those flies that conditionally express lamin B in the muscle tissue or fat body cells or in flies which express kuk in muscle tissue was found (Figure 2E-H).

Thus, the early onset of behavioral decline corresponds to the reduced life span of these flies.

To investigate whether there is an increase in DNA damage in aging flies, 1 week and 5 weeks old muscle tissue was stained with the H2A.X antibody which indicates foci of double-stranded DNA damage. Figure 3A shows DNA damage and gene silencing in adult flies by H2A.X staining in 1 week or 5 weeks old wt longitudinal muscle nuclei. Z-stacks of confocal images were fused to show several nuclei. Position effect variegation (PEV) of the wm4 allele is presented in Figure 3B. In heterozygous kuk mutants (wm4; Δkuk/+) the silencing of wm4 expression is suppressed. In homozygous kuk mutants (wm4; Δkuk/Δkuk) the suppression of PEV is even more pronounced compared to control flies (wm4; +/+). In Figure 3C photometric measurements of the eye pigments of flies from the genotypes described in Figure 3B are plotted. n = 50 males per genotype with error bars ± s.e.m..

In young flies, only occasionally H2A.X foci were present, whereas the number of muscle nuclei with numerous H2A.X foci increased, when the animal became older, indicating more DNA damage (Figure 3A). H2A.X foci accumulated in aging flies, baboons and mice, aged human cells, and cells from patients with HGPS. To find out whether kuk is indeed capable influencing gene silencing or heterochromatic spreading, it was tested whether kuk has an effect on PEV using the wm4 allele, where the white gene is translocated close to the heterochromatic region of the centromere. In heterozygous kuk mutants the silencing of wm4 expression was suppressed, and there were patches of red-pigmented ommatidia in the fly eyes (Figure 3B). In homozygous kuk mutants the suppression of PEV was pronounced, and a high number of ommatidia showed the red eye pigment. These results provide functional evidence that kuk contribute to the regulation of heterochromatin formation.

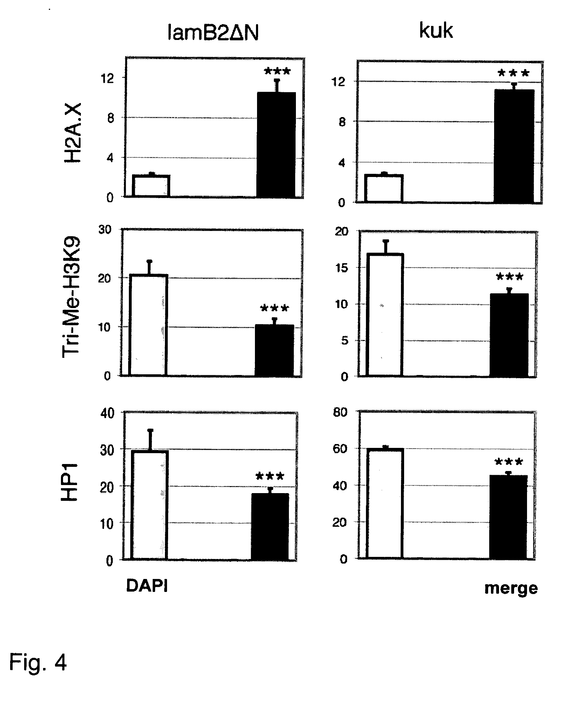

In cultured fibroblasts from aged individuals, in HGPS cells, and in cells overexpressing IaminΔ50 aberrant shaped nuclei, HP1 and Tri-Me-H3K9 staining levels were reduced, and DNA damage marked by H2A.X antibodies was increased. To investigate if the induction of these changes is a lamin specific function or whether unspecific proteins induced age-related phenotypes, kuk or laminBΔN, a truncated lamin B variant, which only consists of a nuclear localization signal and the C-terminal farnesylation site was expressed in mouse fibroblasts. In Figure 4 overexpression of laminBΔN or kuk inducing DNA damage and changing levels of nuclear proteins in cultured mouse fibroblast cells are demonstrated. The total number of H2A.X foci in one focus plane (H2A.X) or the relative fluorescence intensities (%) of laminBΔN or kuk transfected cells and not transfected control cells (Tri-Me-H3K9, HP1) are compared. Tests are two-tailed, mean values of three replicates per experiments are shown with error bars ± s.e.m.. Indeed, the number of H2A.X foci containing nuclei was significantly higher in the group of transfected cells compared to the control group. Moreover, HP1 and Tri-Me-H3K9 staining levels in cells transfected with kuk or laminBΔN compared to non-transfected cells were significantly reduced. Therefore, kuk as well as laminBΔN are able to induce cellular phenotypes similar to cultured fibroblasts from old individuals or cells from HGPS patients. Strikingly, A414 staining, a marker for nucleoporins, strongly increased in laminBΔN or in kuk transfected cells, whereas the control cells showed a normal punctuated nuclear pore staining (Figure 4). Similar to nucleoporin staining of late passage-HGPS cells, nuclear pores aggregated to large bright masses, which were associated with the infoldings of the nuclear envelope of highly lobulated nuclei.

In conclusion, it was shown that a truncated lamin B construct reduced to its nuclear localization and farnesylation signal, and even the *Drosophila* specific kuk are able to induce age-related nuclear changes in mammalian cells.

These studies demonstrate that aging phenotypes as reduced life span, early onset of behavioral decline, reduced heterochromatin levels, and increase in DNA damage is induced by lamin B and kuk.

### References

Bauer et al., Proc. Natl. Acad. Sci. 101, pp. 12980-12985, 2004
Brandt et al., Current Biology 16, pp. 543-552, 2006
Fryberg et al., J. Cell. Biol. 110, 1999-2011, 1990
Gargano et al., Exp. Gerontol. 40, 386-395, 2005
Guillemin et al., Nat. Cell. Biol. 3, pp. 848-851, 2001
Moulson et al., Hum. Mutation 28(9), pp. 882-889, 2007
Prüfert et al., J. Cell Sci. 117, pp. 6095-6104, 2004
Scaffidi and Misteli, Nat. Med. 11, pp. 1059-1063, 2006
Shumaker et al., Proc. Natl. Acad. Sci. 103, pp. 8703-8708, 2006

## Claims

1. A method for screening an age-related candidate substance or an age-related candidate gene comprising the steps of:
a) expressing at least one polypeptide comprising a nuclear localization signal and a farnesylation site using means for genetic expression;
b) applying at least one candidate substance or at least one candidate gene to the means; and
c) measuring at least one aging parameter from the means,
wherein a modification of the aging parameter indicates an age-related substance or an age-related gene.

2. The method of claim 1, wherein the aging parameter is selected from the group consisting of DNA stability and damage, nuclear morphology, chromatin formation, gene expression, behavioral pattern of the means, organelle modification and organelle distribution.

3. The method of claim 1 or 2, wherein the means is selected from the group consisting of prokaryotes; animals; insects, preferably *Drosophila melanogaster*; nematodes, preferably *Caenorhabditis elegans;* plants; fungi, preferably *Saccharomyces cerevisiae*; protists; transgenic vectors; viruses; and cells, preferably insect, amphibian or mammalian cells.

4. The method of any of the preceding claims, wherein the polypeptide is selected from the group consisting of kugelkern, A type lamin and B type lamin, preferably laminBΔN.

5. The method of any of the preceding claims, wherein the lifespan of the means is reduced, preferably up to 60%, more preferably up to 70%.

6. The method of any of the preceding claims, wherein an age-related phenotype of the means appears within 48 h, preferably within 24 h.

7. The method of any of the preceding claims, wherein the polypeptide is conditionally expressed, preferably by a gene switch sequence.

8. An assay for screening age-related candidate substances or age-related candidate genes comprising at least one means for expressing at least one polypeptide and a system for measuring at least one aging parameter, wherein the means comprises a nuclear localization signal and a farnesylation site.

9. The assay of claim 8, wherein the means comprise a conditional expression sequence, preferably a gene switch sequence.

10. The assay of claim 8 or 9, further comprising at least one candidate substance or at least one candidate gene.

11. A nucleic acid encoding a polypeptide comprising a nuclear localization signal, a farnesylation site and a conditional expression sequence.

12. The nucleic acid of claim 11, wherein the polypeptide is selected from the group consisting of kugelkern, A type lamin and B type lamin, preferably laminBΔN.

13. The nucleic acid of claims 11 or 12, wherein the conditional expression sequence is a gene switch sequence, preferably selected from the group consisting of the gene switch-myosin heavy chain sequence, the gene switch-embryonic lethal abnormal vision sequence and the gene switch-S1 106 sequence.

14. Use of the assay of claims 8 to 10 for screening at least one age-related candidate substance or at least one age-related candidate gene.

15. Use of claim 14, wherein the substance is a pharmaceutically active substance for therapeutic and/or prophylactic treatment of age-related disorders.
